# EUROPEAN PATENT SPECIFICATION

(11) **EP 1 265 565 B1**
(45) Date of publication and mention of the grant of the patent: **26.09.2007**
(21) Application number: 01910607.9
(22) Date of filing: 13.02.2001
(51) Int. Cl.: A61F 5/00

(54) **MOLDED ORTHOPAEDIC DEVICES**
GEGOSSENE ORTHOPÄDISCHE VORRICHTUNG
DISPOSITIFS ORTHOPEDIQUES MOULES

(30) Priority: 15.02.2000 US 504980; 02.11.2000 US 704364
(43) Date of publication of application: 18.12.2002
(73) Proprietor: ROYCE MEDICAL COMPANY, Camarillo, California 93012 (US)
(72) Inventor: IGLESIAS, Joseph, M., Thousand Oaks, CA 91320 (US); GRIM, Tracy, E., Tulsa, OK 74137 (US); WYATT, Stacy, Camarillo, CA 93010 (US); PELOTE, Steven, T., Valley Village, CA 91607 (US); TRAN, Luis, F., North Hills, CA 91343 (US)
(74) Representative: Hanna, Peter William Derek
(86) International application number: PCT/US2001/004587
(87) International publication number: WO 2001/060289

(56) References cited:
- US-A- 4 854 310
- US-A- 5 286 249
- US-A- 5 368 549
- US-A- 5 584 799
- US-A- 5 713 837
- US-A- 5 772 620
- US-A- 5 782 784

## Description

### BACKGROUND OF THE INVENTION

### A. Field of the Invention

The present invention relates to orthopaedic supports and, more particularly, to an orthopaedic support that has a molded exostructure.

### B. Prior Art

There are a number of known ways to stiffen fabric orthopaedic supports for injured parts of the anatomy. U. S. Patent No. 4,724,847, for example, discloses an ankle brace that has a plurality of pockets. Rigid stay members are inserted into the pockets to form a rigid structure that surrounds and immobilizes the ankle. U. S. Patent Nos. 3,298,365, 4,280,488, 4,825,856, and 4,440,158, among others, disclose similar arrangements.

A drawback of these designs is that they require a great deal of labor to construct. Workers must be hired to cut many separate pieces of fabric, sew the supports together, insert the rigid stays and so on. A further drawback is that the stays are typically die-cut from plastic of constant thickness. The shape of the stays is therefore quite limited, and the final support often does not fit the anatomy perfectly.

While stays can be manufactured to have a particular contour, the manufacturing process is not simple and is often fairly expensive.

Efforts have been made outside of the orthopaedic support art to create stiffened, custom-shaped objects by injecting hardenable material into a mold in which fabric has been placed. For example, European Patent Application No. 89103277.3, which the EPO published on February 24, 1989 as publication number 0 332 899, discloses a diaphragm formed by injection molding plastic onto a piece of fabric. The diaphragm acts as a pressure barrier in an automobile engine. European Patent Application No. 87101406.4, published on February 3, 1987 as publication no. 0 234 341, discloses creating fiber reinforced structures for automobiles. Fibrous material is placed into a mold, and then resin is injected into the mold. The resin saturates the fabric and eventually sets, thereby forming a reinforced automobile part. Other examples include U. S. Patent Nos. 5,093,067 and 5,456,976.

U. S. Patent No. 5,647,150, which issued July 15, 1997, discloses forming a shoe by stretching a sock about a mold. A thermoplastic film layer is positioned between the mold and the fabric layer. Thermoplastic material is then allowed to flow through the fabric and bond with the thermoplastic film layer, thereby securing the thermoplastic material to the fabric. However, the method requires that the thermoplastic material flow entirely through the fabric in order to bond with a thermoplastic film layer on the opposite side of the fabric.

The state of the art according to US-A-5 713 837 is acknowledged in the preamble of claim 1.

### SUMMARY OF THE INVENTION

The object of the present invention is to advance the art with respect to orthopaedic supports.

The present invention provides an orthopaedic wrist support in accordance with claims which follow. Generally speaking, the present invention is an orthopaedic support having a flexible inner member and a molded exo-skeleton.

In particular, the invention provides an orthopaedic wrist support comprising:
a molded plastic exostructure supplying support for resisting motion of said wrist;
said molded plastic exostructure comprising a web bridge portion that is adapted to extend across the web of the hand, said web bridge portion partially defining a thumb opening;
said web bridge portion comprising a padded, flexible member extending about at least a portion of said web bridge portion to provide cushioning for the web of the hand, or a flexible fabric that has been that has been molded or secured into place on the exostructure,
characterized in that said web bridge portion is pivotally attached to said exostructure.

Another aspect of the invention relates to an orthopaedic support having an attachable stay. A versatile wrist support comprises a molded plastic exostructure supplying support for resisting motion of said wrist. An inner fabric support is attached to said molded exostructure for providing cushioning to the wrist area. A separate, attachable stay for optional attachment to said molded exostructure is provided to add further rigidity to the exostructure. The wrist support has a first relatively flexible mode in which the attachable stay is not attached to the wrist support, and a second relatively stiff mode in which the attachable stay is attached to the wrist support.

In particular embodiments, the stay may be formed from the group of materials constituting aluminum, steel and molded plastic. The stay may be a bendable aluminum stay. The support may also have a third mode in which said aluminum stay has been bent after attachment to the support in order to alter the shape of the support.

The support may include a molded recess for receiving said stay.

The support may comprise a molded plastic exostructure supplying support for resisting motion of said wrist. Said molded plastic exostructure may comprise said web bridge portion that is adapted to extend across the web of a hand. A padded, flexible member extends about at least a portion of said web bridge portion to provide cushioning for the web of the hand. The molded plastic exostructure may optionally include a molded recessed area for receiving said padded member.

Another aspect of the invention is an adjustable wrist support having an adjustable forearm portion that can accommodate various sizes of forearms. One such embodiment includes a molded plastic exostructure supplying support for resisting motion of said wrist. An inner cushion is attached to said molded exostructure for providing cushioning to the wrist area. The exostructure has a forearm portion and a hand portion. The forearm portion includes an adjustable closure, wherein the forearm portion may be adjusted to fit the forearms of a variety of different users.

In various embodiments, the adjustable closure comprises a strap having hook and-loop material. The adjustable closure may comprise a first and second strap, said first strap having a plurality of posts and said second strap having a plurality of holes to receive said posts. The adjustable closure may alternatively comprise a strap secured to said exostructure and a clip adjustably secured to said strap, said clip having an aperture, said exostructure having a hook to which said clip secures.

It is noted that the present invention encompasses a wide variety of different orthopaedic supports, including splints, such as volar or dorsal splints. The splints may be unitarily molded, and may include a thumbhole and a portion for extending across the web of the hand.

Another aspect of the present invention is a support having an injection-molded exostructure and interior padding. The interior padding is provided with a support structure that is molded onto the padding. The support structure may be bonded to the exostructure to form a padded orthopaedic support. In one embodiment of the invention, the support structure of the interior padding member is attached to said exostructure by a method selected from the group constituting ultrasonic welding, snaps, hook-and-loop material, rivets or an adhesive.

In various embodiments of the present invention, the thickness of the exosupport may be increased or decreased in certain areas, to provide different levels of support and flexibility at different points on the support. The exostructure may be adapted to receive a separate support member after molding. Also, the exostructure may be molded over a stay or other member that is attached to the flexible material or otherwise introduced into the mold prior to injection molding.

The support may further comprise straps about which the exostructure is injection molded, thereby attaching the straps to the support. The support may include one or more bladders, and may also include a pump for inflating the bladders.

Alternatively, the support may include gel or foam pads.

The exostructure may be molded from more than one type of material. For example, the exostructure may be a more flexible material in one region where flexibility is desired, and a stiffer material in another region where greater stiffness is desired.

Other objects and features of the invention will become apparent from a review of the Detailed Description below, from the drawings, and from the claims.

### BRIEF DESCRIPTION OF THE DRAWINGS

Fig. 1 shows an orthopaedic wrist support, in which an exostructure is molded in a three-dimensional shape inside of a mold;
Fig. 2 illustrates a finished orthopaedic wrist support incorporating the molded exostructure of Fig. 1;
Fig. 3 illustrates an alternative molded exostructure;
Fig. 4 illustrates a brace formed from the molded exostructure of Fig. 3;
Fig. 5 is a cross-sectional view taken about line 22-22 of Fig. 4;
Fig. 6 is an illustration of a configurable molded brace;
Figs. 7 and 8 are components that can be added onto the brace of Fig. 6 in order to form a thumb support;
Figs. 9A and 9B illustrate an arrangement for snapping the components of Figs. 7 and 8 unto the base brace of Fig. 6;
Fig. 10 illustrates a molded brace in which an optional stay can be attached to the brace for further support;
Fig. 11 illustrates a two-part brace in which the soft goods inner liner includes its own plastic shell;
Fig. 12 illustrates an alternative brace in which resilient padding covers an indented portion of the web bridge portion of the brace;
Fig. 13 is a further molded wrist support;
Fig. 14 illustrates the wrist support of Fig. 13 with additional padding arranged in the web area of the thumb support;
Figs. 15 and 16 illustrate an alternative attachment arrangement in which an adjustable baseball cap type securing system is utilized;
Fig. 17 illustrates a further alternative embodiment having a molded exostructure that is formed in separate parts and then is joined together after molding;
Fig. 18 illustrates an embodiment of a brace ***in accordance with the present invention*** having a pivoting bridge for extending across the web portion of the hand;
Fig. 19 illustrates the use of generally "S"-shaped junctions where the web bridge portion of a wrist support connects at either end with the adjacent support pieces; and
Fig. 20 illustrates the use of generally "S"-shaped junctions at the bottom of the thumb opening of the brace.

### DETAILED DESCRIPTION OF THE PREFERRED EMBODIMENTS

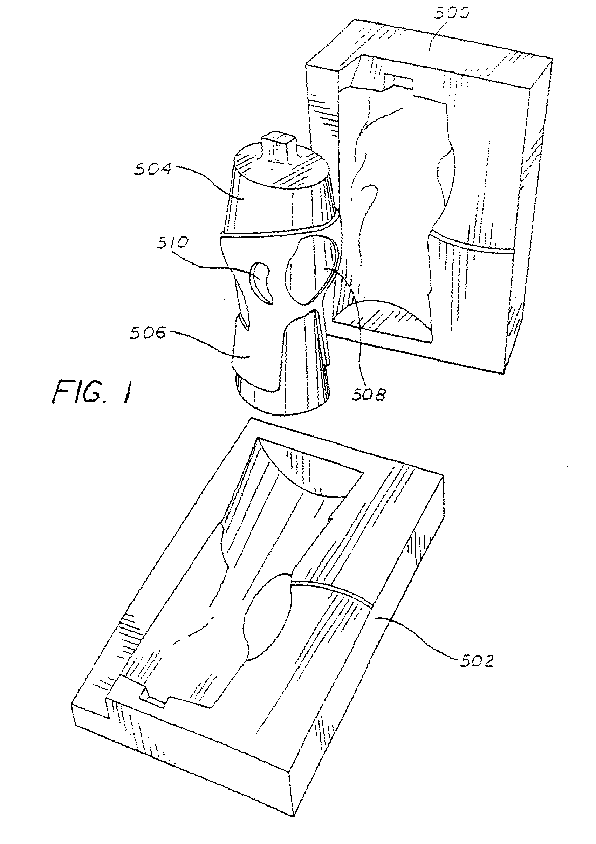

Figure 1 illustrates an apparatus for forming an injection molded wrist brace.

The wrist brace may optionally have an incorporated stay and a unitarily molded web capturing area. The brace may incorporate thick and thin areas in different portions of the brace, in order to provide additional support in some areas and greater flexibility in other areas. The method of manufacturing the brace includes molding an exostructure about a three-dimensional core, rather than molding the plastic onto a relatively flat piece of material and then folding it up into a brace.

In one method of manufacturing such a brace, a stockinet or tubular soft good padding member is extended about the three-dimensional core.

Plastic is then molded about the core and into the soft good material, to form a unitary, padded support. In an alternative method of manufacturing the brace, the exostructure is molded first about the three-dimensional mold core. The soft good padding is then attached separately to the wrist-shaped molded exostructure. Considering Figure 1 in some detail, a mold includes a first mold piece 500, a second mold piece 502 and a mold core 504. In Figure 18, an exostructure 506 has been molded about the mold core 504. The molded exostructure includes a thumb hole 508 and an optional aperture 510 for relieving pressure in a particular portion of the wrist.

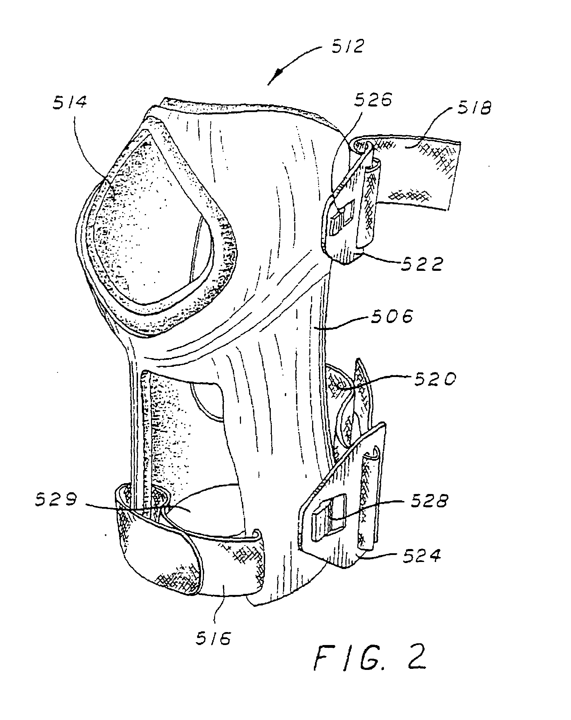

Figure 2 illustrates the exostructure 506 as it appears on a finished wrist support 512. A soft padding interior has been attached to the exostructure 506. The soft padding interior 514 is attached to the exostructure 506 after the exostructure 506 has been molded. The soft padding 514 can be attached to the exostructure 506 with an adhesive, such as a heat-activated or urethane-based adhesive, or by other known attachment means, such as rivets, hook and loop type fasteners, or even molded directly to the exostructure. The pad may also be attached using other heating means, and may even be formed at the same time it is bonded to the exostructure.

A radial forearm adjustment strap 516 includes hook and loop type material so that the forearm portion of the brace may be adjusted on the user. This adjustment strap 516 is a mechanism that permits the manufacturer to make a smaller number of different sized braces to fit the wide range of different sized users. Experience has shown that the diameter of the forearm can vary greatly among different people, even between people who have approximately the same sized hands. By making the forearm portion of the brace adjustable in size, one brace can be made to fit a wide variety of users.

Figure 2 also illustrates additional closure straps 518 and 520 for further securing the brace to the wrist and forearm. Each of straps 518 and 520 include respective clasps 522 and 524. The clasps hook onto hooks 526 and 528, respectively, to secure the support to the wrist and forearm.

Another feature of Fig. 2 is an opening 529 in which there is no material. The purpose of the opening 529 is to provide pressure relief in the region of the arm often associated with carpal tunnel syndrome. The pressure relief opening 529 prevents pressure that could result if that portion of the arm swelled against the material of the brace. The pressure relief opening 529 facilitates recovery from carpal tunnel syndrome, and may also help to prevent the occurrence of carpal tunnel syndrome. Alternatively, the pressure relief opening 529 may be filled in with a mesh or other relatively flexible material, yet still provide pressure relief.

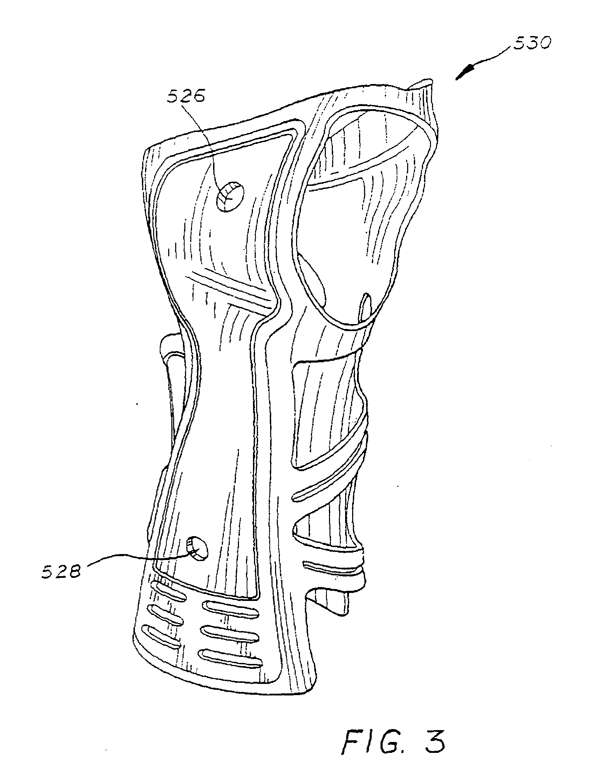

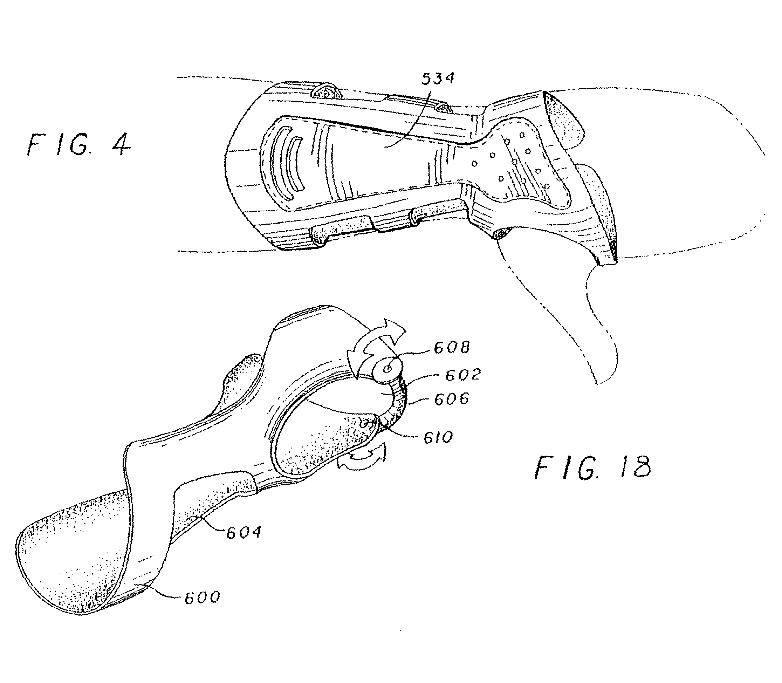

Figure 3 illustrates another molded exostructure 530. Figure 4 illustrates the exostructure of Figure 3 having been completed into an orthopaedic wrist support. In this support, an aluminum stay 534 is molded into the exostructure itself. The aluminum stay provides support for the forearm and wrist structure, and typically permits a doctor or other user to bend the support somewhat to customize the shape of the support for a particular user. To manufacture the support of Figure 4, a thin contoured aluminum stay is placed in a mold arrangement similar to that illustrated in Figure 1. The stay is held in place by pins within the mold (not shown), which explains the apertures 526 and 528 in Figure 3. As can be appreciated by those skilled in the art, the aluminum stay 534 can alternatively be made of other materials, such as steel. Other materials may be used, depending on the desired stiffness or flexibility of the support. Various types of stays, such as palmer and dorsal stays, may be incorporated.

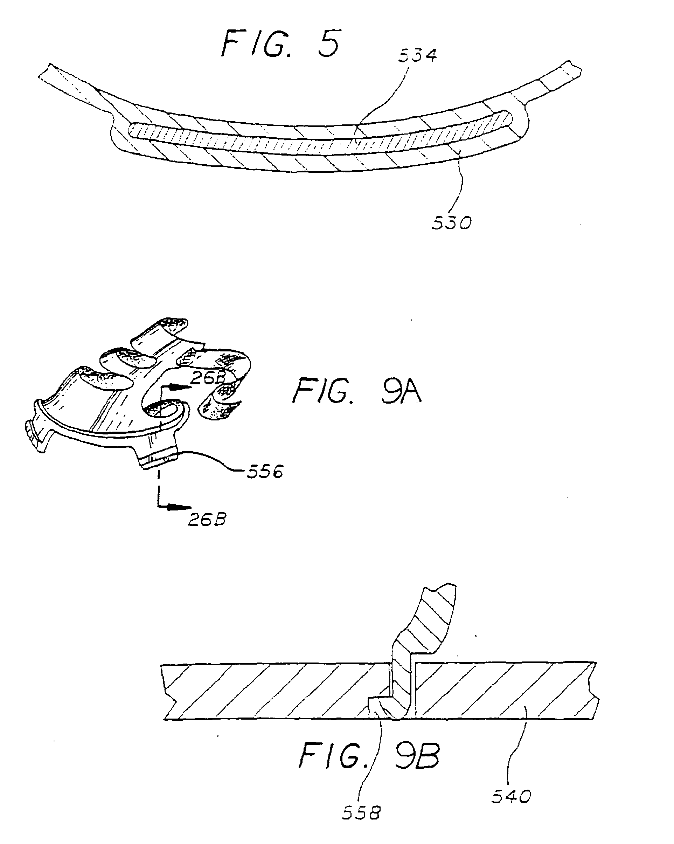

Figure 5 is a sectional view taken about section 22-22 in Figure 4. Figure 5 illustrates the aluminum stay 534 embedded within a portion of the molded exostructure 530.

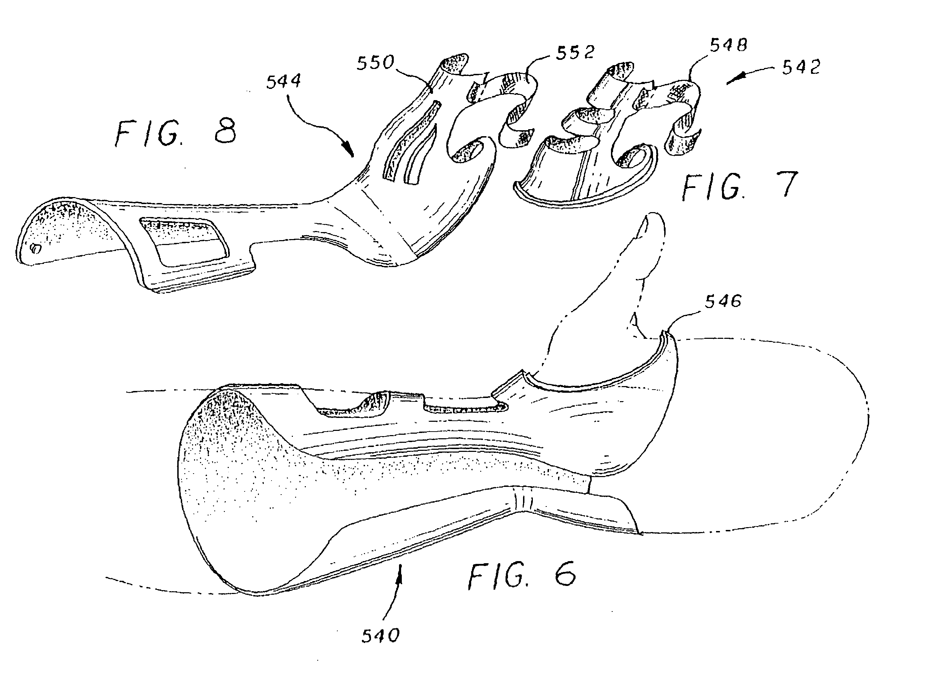

Figure 6 illustrates a brace 540 which may be further configured with optional additional components, such as those illustrated in Figures 7 and 8. Figure 7 illustrates a thumb splint 542 that can be optionally attached to the brace 540 at thumb hole 546. The thumb splint 542 includes a strap 548 for securing the thumb within the splint. The strap 548 may have an end portion with hook and loop type material, and a mating piece of hook and loop material may be adhered to the side of the thumb splint 542, at which the strap 548 may attach.

If the user desires greater support and reinforcement for the orthopaedic support 540, a member 544 such as that shown in Figure 8 may be coupled to the support 540. The component 544 includes a thumb support splint 550, and a support strap 552 for securing the thumb within the splint. Either splint 542 or 544 may be attached to support 540 by way of adhesive, ultrasonic bonding, rivets, snaps, or other means known in the art for securing member to one another. An advantage of the arrangement illustrated in Figures 6 through 8 is that a single support 540 may serve as the base for a number of different wrist supports.

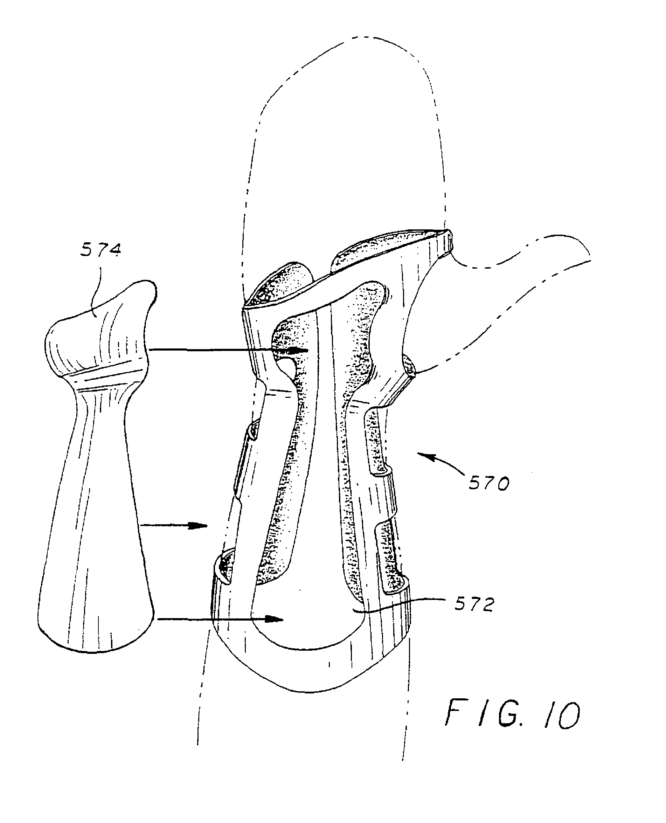

Figure 9A and 9B shows that the component 542 may be snapped into place in one support of the system of Figures 6 through 8. In the support of Figure 9A, the thumb splint 542 is provided with snaps such as 556. The snap hook members such as 556 engage with apertures such as 558 in Figure 9B to enable the splint 542 to snap into place on the brace 540 in Figure 9b.

Figure 10 illustrates a further orthopaedic wrist support 570 having an indentation 572 to receive an optional stay 574. The brace can be used without the optional stay 574, but the stay 574 may be added to the brace to further stiffen the brace, when desired. The stay 574 may be made of any of a variety of materials, such as aluminum, steel or a molded polymer. When thin aluminum or other metals are used, the doctor or end-user may bend stay 574 to alter the shape of the support for custom fitting. A molded polymer stay, however, is less expensive and may be equally effective for stiffening the support in specific circumstances.

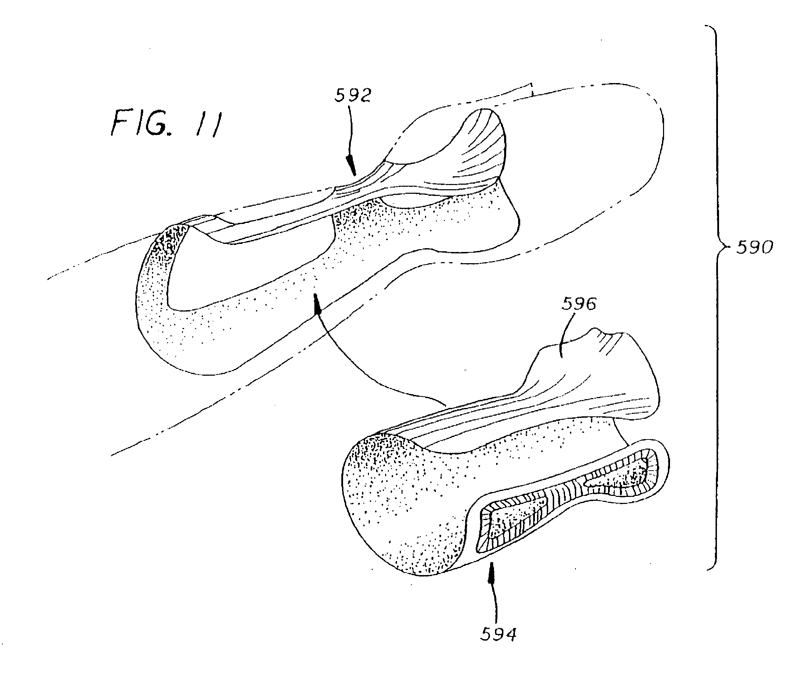

Figure 11 illustrates a two-part support 590, which includes an exterior exostructure 592 and a padded soft goods member 594. The padded soft goods member 594 may have a molded exostructure of its own 596. The function of the exostructure 596 is to provide additional support to the wrist along the ulnar side, while not circumferentially surrounding the hand with plastic to do so. The entire unit 594 is manufactured separately from member 592 and then is attached by means of ultrasonic bonding, adhesive, snaps, hook and loop material fasteners, rivets or other fastening means known in the art.

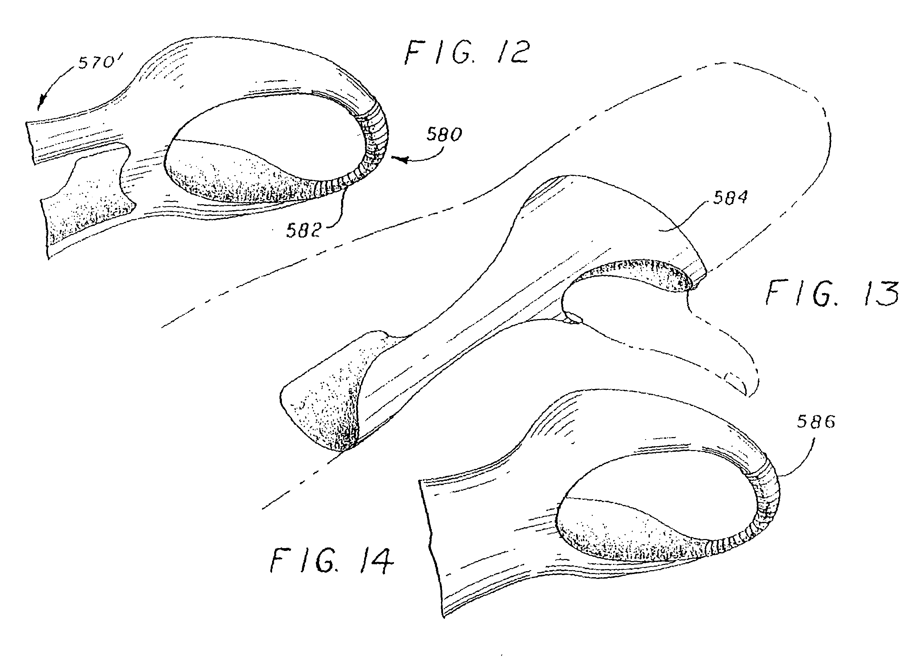

Figure 12 illustrates the portion 580 of the support 570 that extends across the web area between the thumb and the forefinger. This portion 580 may be referred to as a web portion bridge, because it extends across the web portion of the hand. In this particular support, a soft overmold 582 is molded onto the portion 580 to provide padding for the web area of the hand. The advantage of the overmold 582 is that there is no hard plastic to dig into the web area of the hand. The molded polymer portion of the support retains the shape of the web area 580, while the overmold provides the cushioning. As a result, the web area 580 does not stretch or otherwise become misshapen.

Figure 13 illustrates an alternative brace in which less material is used on the upper and lower portions of the wrist, and a greater quantity of material is used along the side of the wrist. Although means for support are not illustrated in Figure 13, the various other means illustrated in other Figures may be used, such as hook and loop material fasteners, straps, snap-straps, and various other means. Figure 14 illustrates a concept similar to that shown in Figure 12, wherein a soft overmold is placed in the web area of the support. The brace of Fig. 14 has an indentation such that when the soft good over-mold is formed over the indentation, the overmold is flush with the rest of the molded brace, or the difference in thickness is at least reduced.

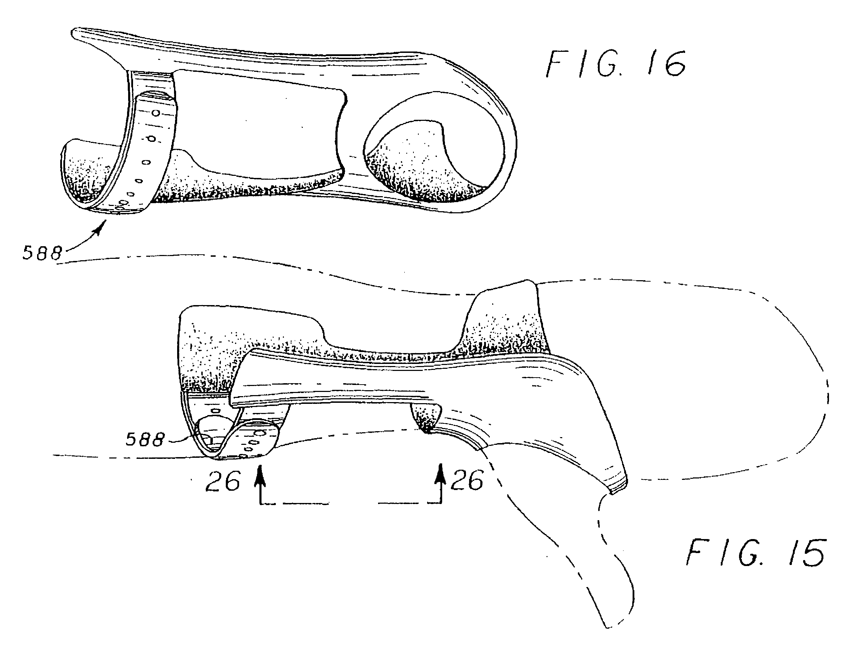

Figures 15 and 16 illustrate a further means for adjusting the forearm portion of the support. This particular means for adjusting the forearm portion of the brace 588 is similar to baseball cap-style adjustment means, which allow the user to expand or contract the distance between two members. The adjustment mechanism consists of a first member having a plurality of spaced posts, and a mating member having a plurality of apertures with openings sized such that the posts may pass through the openings under pressure from the user, but will not withdraw from the apertures without significant pressure. This type of fastening mechanism is widely known and is used extensively on baseball caps.

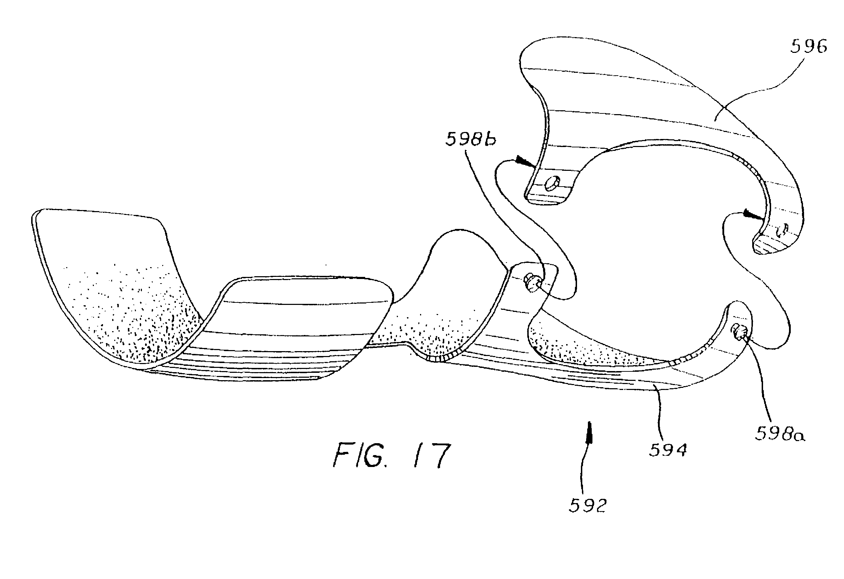

Figure 17 illustrates a wrist support 592 having a first piece 594 and a second mating piece 596. Unlike other supports described in which the entire support is formed in a single molding step, the support 592 of Figure 17 can be formed in separate pieces in separate molding steps. After molding is completed, the pieces 594 and 596 are joined together by way of snaps 598A and 598B or by alternative joining means such as ultrasonic bonding, adhesives, rivets, or other means known in the art. Although Figure 17 illustrates a brace 592 in which there are just two members that are joined together to form the completed brace, it may be made of multiple additional components that are formed separately and then joined together after molding. The brace 592 may be supplemented with padding, additional supports, or other components to stiffen, strengthen or otherwise improve the function of the brace. The system of Fig. 17 also allows for construction of a variety of different braces using, for example, a standard piece 594 and a selection of different pieces 596. The piece 596 may be chosen from the selection of different pieces to construct a support having characteristics or configuration that is desired.

Figure 18 illustrates ***an embodiment of the present invention,*** in which a support has an exostructure 600 with a thumbhole 602 and padding 604. A web bridge 606 is provided to extend across the web portion of the hand. In this embodiment, the web bridge is pivotally mounted at hinge points 608 and 610, so that the web bridge can pivot as the web of the hand moves back and forth relative to the web bridge. The hinge points 608 and 610 can be any of a variety of different mechanical hinges known in the art.

Embodiments of the wrist brace may have a thumbhole, with an overmold about the thumbhole. The overmold may be of a softer, more resilient material than the exostructure itself, to help cushion the thumb from the exostructure. Similarly, overmolds may be applied about other openings in the wrist brace or on other types of supports for various purposes. The bridge portion extending across the web of the hand may be an overmolded material.

Embodiments of the present invention may include overmolding in certain areas to provide a non-skid surface. For example, in wrist support embodiments, the wrist support may have a palm section with an overmold on at least a portion of the palm section. The overmold provides a non-skid surface, to reduce the tendency of the palm portion from sliding on surfaces such as a table top or other surfaces on which the palm may come into contact.

In another variation, in embodiments in which a support is supplemented with a padding member that has its own plastic shell, the padding member does not necessarily need to be bonded to the exostructure, but may be attached in some other way, such as by rivets, hooks, hook-and-loop material, snaps or other attachment methods known in the art. The plastic structure that is molded onto the padding may serve to provide support to the anatomy that the exostructure does not otherwise provide.

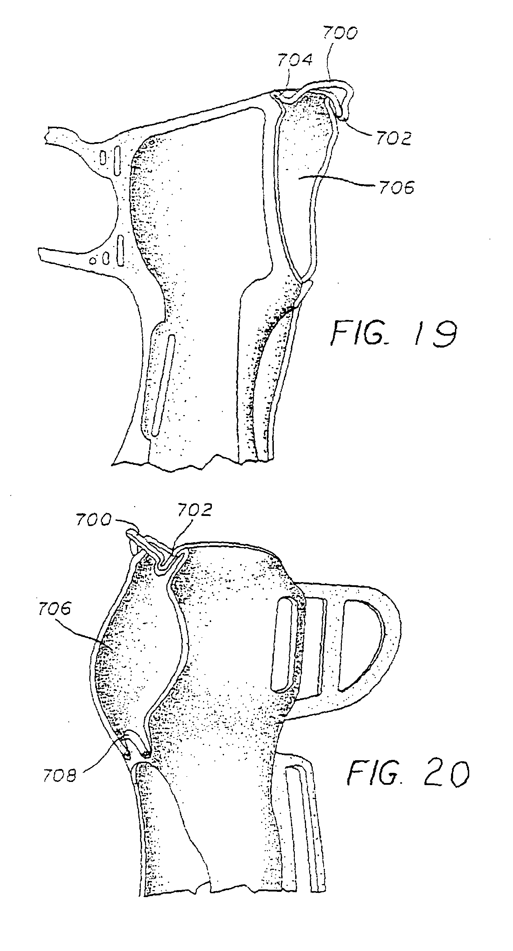

It is also possible to provide a support according to the present invention with arrangements to provide additional flexibility in specific areas. For example, Figures 19 and 20 illustrate how specific portions of the support may be provided with indented areas to increase flexibility. In Fig. 19, a portion 700 to extend across the bridge of the hand is joined at either end by indented, "S"-shaped junctions 702 and 704, respectively. The junctions and the portion 700 are unitarily molded with the adjoining portions of the brace. The generally "S"-shaped junctions allow the portion 700 to flex easily. In Fig. 19, reference numeral 706 refers to the thumb opening in the brace. The indented areas 702,704 may have an "S" shape, or other indented shape. Areas of the support in which this arrangement may be used to advantage include the portion of the support in which a web bridge meets the body of the brace, as well as in the thenar bridge, which is the area across the base of the thumb. Fig. 20 illustrates one such "S" shaped junction at the bottom of the thumb opening 706. The indented areas can be covered with an overmold, to provide particularly comfortable areas on the support.

In another wrist brace embodiment of the invention, a molded plastic exostructure may be covered with an overmold on at least any edges where digital motion of the hand causes the skin to contact the edges of the exostructure. The overmold provides comfort to the user.

Accordingly, the present invention is not limited precisely to the arrangements as shown in the drawings and as described in detail hereinabove.

## Claims

1. An orthopaedic wrist support comprising:
a molded plastic exostructure (600) supplying support for resisting motion of said wrist;
said molded plastic exostructure comprising a web bridge portion that is adapted to extend across the web of the hand, said web bridge portion partially defining a thumb opening (602);
said web bridge portion comprising a padded, flexible member (606) extending about at least a portion of said web bridge portion to provide cushioning for the web of the hand, or a flexible fabric that has been molded or secured into place on the exostructure,
**characterized in that** said web bridge portion is pivotally attached (608,610) to said exostructure (600).

2. An orthopaedic wrist support according to claim 1, wherein said exostructure comprises a molded outer exostructure (600) and a softgoods lining (604) in said exostructure.

3. An orthopaedic wrist support according to claim 1, wherein said exostructure comprises a stay (534,574) formed from aluminium, steel or molded plastic.

4. An orthopaedic wrist support according to claim 3, wherein said stay (534,574) is a bendable aluminium stay.

5. An orthopaedic wrist support according to claim 4, in which said aluminium stay (534,574) has been bent after attachment to the support in order to alter the shape of the support.

6. An orthopaedic wrist support according to claim 3, wherein said stay (534,574) is a dorsal stay or a palmer stay.

7. An orthopaedic wrist support according to claim 1, wherein said member comprises an overmolded material.

8. An orthopaedic wrist support according to claim 1, wherein said padded flexible member (606) is attached to said exostructure (600) by ultrasonic welding, a snap, hook-and-loop material, a hook, a rivet or an adhesive.

9. An orthopaedic wrist support according to claim 1, wherein said support is a volar splint or a dorsal splint.

10. An orthopaedic wrist support according to claim 1, wherein said support comprises a volar plate and a dorsal plate, said web bridge portion (606) extending between said volar plate and said dorsal plate.

11. An orthopaedic wrist support according to claim 10, wherein said web bridge portion (606) is pivotally attached (608,610) at an end of the web bridge to at least one of said volar plate and said dorsal plate.

12. An orthopaedic wrist support according to claim 11, wherein said web bridge portion (606) maintains said volar and said dorsal plates a set distance from one another.

13. An orthopaedic wrist support according to preceding claim, wherein said exostructure (600) at least partially surrounds the portion of the anatomy to be supported.

14. An orthopaedic wrist support according to any preceding claim, wherein said exostructure (506,530) has a forearm portion and a hand portion, said forearm portion including an adjustable radial forearm strap (516,520,588) wherein the forearm portion may be adjusted to fit the forearms of a variety of different users.

15. An orthopaedic wrist support according to claim 14, wherein said adjustable radial forearm strap comprises a strap
- having hook and loop material (516);
- comprising a first and a second strap (588), said first strap having a plurality of posts and said second strap having a plurality of holes to receive said posts; or
- is a closure strap (520) secured to said exostructure and a clip (524) adjustably secured to said strap, said clip having an aperture, said exostructure (506) having a hook (528) to which said clip secures.

16. An orthopaedic wrist support according to any preceding claim, wherein said exostructure (600) comprises plastics of different densities.

17. An orthopaedic wrist support according to any preceding claim, wherein the thickness of said exostructure (600) is non-uniform to provide different levels of support at different points on the support.

18. An orthopaedic wrist support (590,592) according to any preceding claim, wherein said molded plastic exostructure comprises first and second separate pieces.

19. An orthopaedic wrist support according to claim 18, wherein said first and second separate pieces (594,596) are attached to one another by a method selected from ultrasonic welding, snaps (598a,598b), hook-and-loop material, rivets or an adhesive.

20. An orthopaedic wrist support according to any preceding claim, wherein said molded exostructure (600) is directly molded onto said fabric within a mold.

21. An orthopaedic wrist support according to any preceding claim, wherein the support has a carpal tunnel pressure relief opening (529).

22. An orthopaedic wrist support according to any preceding claim, wherein the support has a palm portion, with an overmold on at least a part of the palm portion.

## Patentansprüche

1. Orthopädische Handgelenkstütze, umfassend:
eine formgegossene Kunststoff-Außenstruktur (600), die eine Stützung zum Widerstehen einer Bewegung des Handgelenks bietet;
wobei die formgegossene Kunststoff-Außenstruktur einen Gewebebrückenabschnitt umfasst, der angepasst ist, sich entlang des Gewebes der Hand zu erstrecken, wobei der Gewebebrückenabschnitt teilweise eine Daumenöffnung (602) definiert;
wobei der Gewebebrückenabschnitt ein gepolstertes, flexibles Element (606) umfasst, das sich um wenigstens einen Abschnitt des Gewebebrückenabschnitts erstreckt, um eine Abpufferung für das Gewebe der Hand bereitzustellen, oder einen flexiblen Stoff umfasst, der an der Außenstruktur passend formgegossen oder gesichert ist,
**dadurch gekennzeichnet, dass** der Gewebebrückenabschnitt an der Außenstruktur (600) schwenkbar angebracht ist.

2. Orthopädische Handgelenkstütze nach Anspruch 1, wobei die Außenstruktur eine formgegossene äußere Außenstruktur (600) und ein Textilfutter (604) in der Außenstruktur umfasst.

3. Orthopädische Handgelenkstütze nach Anspruch 1, wobei die Außenstruktur eine Strebe (534, 574) umfasst, die aus Aluminium, Stahl oder einem formgegossenen Kunststoff gebildet ist.

4. Orthopädische Handgelenkstütze nach Anspruch 3, wobei die Strebe (534, 574) eine biegbare Aluminiumstrebe ist.

5. Orthopädische Handgelenkstütze nach Anspruch 4, bei der die Aluminiumstrebe (534, 547) nach einem Anbringen an der Stütze gebogen wurde, um die Form der Stütze zu verändern.

6. Orthopädische Handgelenkstütze nach Anspruch 3, wobei die Strebe (534, 574) eine Dorsal-Strebe oder eine Handflächen-Strebe ist.

7. Orthopädische Handgelenkstütze nach Anspruch 1, wobei das Element ein umgossenes Material umfasst.

8. Orthopädische Handgelenkstütze nach Anspruch 1, wobei das gepolsterte, flexible Element (606) an der Außenstruktur (600) durch Ultraschallschweißen, eine Schnappvorrichtung, ein Klettverschlussmaterial, einen Haken, eine Niete oder einen Klebstoff angebracht ist.

9. Orthopädische Handgelenkstütze nach Anspruch 1, wobei die Stütze ein hohlhandseitiger Splint oder ein dorsaler Splint ist.

10. Orthopädische Handgelenkstütze nach Anspruch 1, wobei die Stütze eine hohlhandseitige Platte oder eine dorsale Platte umfasst, wobei sich der Gewebebrückenabschnitt (606) zwischen der hohlhandseitigen Platte und der dorsalen Platte erstreckt.

11. Orthopädische Handgelenkstütze nach Anspruch 10, wobei der Gewebebrückenabschnitt (606) an einem Ende der Gewebebrücke an wenigstens einer der hohlhandseitigen Platte und der dorsalen Platte schwenkbar angebracht (606, 610) ist.

12. Orthopädische Handgelenkstütze nach Anspruch 11, wobei der Gewebebrückenabschnitt (606) die hohlhandseitige und die dorsale Platte in einem eingestellten Abstand voneinander hält.

13. Orthopädische Handgelenkstütze nach einem der vorhergehenden Ansprüche, wobei die Außenstruktur (600) den Abschnitt der zu stützenden Anatomie wenigstens teilweise umgibt.

14. Orthopädische Handgelenkstütze nach einem der vorhergehenden Ansprüche, wobei die Außenstruktur (506, 530) einen Unterarmabschnitt und einen Handabschnitt hat, wobei der Unterarmabschnitt ein einstellbares radiales Unterarmband (516, 520, 588) umfasst, wobei der Unterarmabschnitt eingestellt werden kann, um auf die Unterarme einer Vielzahl von verschiedenen Benutzern zu passen.

15. Orthopädische Handgelenkstütze nach Anspruch 14, wobei das einstellbare radiale Unterarmband ein Band
**·** mit einem Klettverschlussmaterial (516) umfasst;
**·** umfassend ein erstes und zweites Band (588), wobei das erste Band eine Vielzahl von Pfosten hat und das zweite Band eine Vielzahl von Löchern hat, um die Pfosten aufzunehmen; oder
**·** ein Verschlussband (520) ist, das an der Außenstruktur und einem Clip (524) gesichert ist, der an dem Band einstellbar gesichert ist, wobei der Clip eine Öffnung hat, wobei die Außenstruktur (506) einen Haken (528) hat, an dem der Clip gesichert wird.

16. Orthopädische Handgelenkstütze nach einem der vorhergehenden Ansprüche, wobei die Außenstruktur (600) Kunststoffe von verschiedenen Dichten umfasst.

17. Orthopädische Handgelenkstütze nach einem der vorhergehenden Ansprüche, wobei die Dicke der Außenstruktur (600) nicht-einheitlich ist, um verschiedene Niveaus der Stützung an verschiedenen Punkten an der Stütze bereitzustellen.

18. Orthopädische Handgelenkstütze (590, 592) gemäß einem der vorhergehenden Ansprüche, wobei die formgegossene Kunststoff-Außenstruktur erste und zweite getrennte Stücke umfasst.

19. Orthopädische Handgelenkstütze nach Anspruch 18, wobei die ersten und zweiten getrennten Stücke (594, 596) durch ein Verfahren aneinander angebracht sind, das aus Ultraschallschweißen, Schnappvorrichtungen (598a, 598b), einem Klettverschlussmaterial, Nieten oder einem Klebstoff ausgewählt ist.

20. Orthopädische Handgelenkstütze nach einem der vorhergehenden Ansprüche, wobei die formgegossene Außenstruktur (600) innerhalb einer Gussform direkt auf den Stoff formgegossen ist.

21. Orthopädische Handgelenkstütze nach einem der vorhergehenden Ansprüche, wobei die Stütze eine Carpaltunnel-Drucklinderungsöffnung (529) hat.

22. Orthopädische Handgelenkstütze nach einem der vorhergehenden Ansprüche, wobei die Stütze einen Handflächenabschnitt mit einer Umgießung an wenigstens einem Teil des Handflächenabschnitts hat.

## Revendications

1. Support orthopédique de poignet comprenant:
une exostructure en plastique moulé (600) fournissant un support pour le mouvement de résistance dudit poignet ;
ladite exostructure en plastique moulé comprenant une partie de pont sur la paume qui est adaptée pour s'étendre à travers la paume de la main, ladite partie de pont sur la paume définissant partiellement une ouverture pour le pouce (602) ;
ladite partie de pont sur la paume comprenant un élément flexible, rembourré (606) s'étendant au moins autour d'une partie de ladite partie de pont sur la paume pour fournir un rembourrage pour la paume de la main, ou un tissu flexible qui a été moulé ou fixé en place sur l'exostructure,
**caractérisé en ce que** ladite partie de pont sur la paume est fixée de manière pivotante (608, 610) sur ladite exostructure (600).

2. Support orthopédique de poignet selon la revendication 1, dans lequel ladite exostructure comprend une exostructure externe moulée (600) et un revêtement de tissus (604) dans ladite exostructure.

3. Support orthopédique de poignet selon la revendication 1, dans lequel ladite exostructure comprend un tuteur (534, 574) formé à partir d'aluminium, d'acier ou de plastique moulé.

4. Support orthopédique de poignet selon la revendication 3, dans lequel ledit tuteur (534, 574) est un tuteur en aluminium pouvant être plié.

5. Support orthopédique de poignet selon la revendication 4, dans lequel ledit tuteur en aluminium (534, 574) a été plié après avoir été fixé au support afin de modifier la forme du support.

6. Support orthopédique de poignet selon la revendication 3, dans lequel ledit tuteur (534, 574) est un tuteur dorsal ou un tuteur palmaire.

7. Support orthopédique de poignet selon la revendication 1, dans lequel ledit élément comprend un matériau surmoulé.

8. Support orthopédique de poignet selon la revendication 1, dans lequel ledit élément flexible rembourré (606) est fixé à ladite exostructure (600) par soudage par ultrasons, une fermeture par pression, un matériau d'attache à velcro, un crochet, un rivet ou un adhésif.

9. Support orthopédique de poignet selon la revendication 1, dans lequel ledit support est une attelle palmaire ou une attelle dorsale.

10. Support orthopédique de poignet selon la revendication 1, dans lequel ledit support comprend une plaque palmaire ou dorsale, ladite partie de pont sur la paume (606) s'étendant entre ladite plaque palmaire et ladite plaque dorsale.

11. Support orthopédique de poignet selon la revendication 10, dans lequel ladite partie de pont sur la paume (606) est fixée de manière pivotante (608, 610) par une extrémité du pont sur la paume à au moins l'une de ladite plaque palmaire et de ladite plaque dorsale.

12. Support orthopédique de poignet selon la revendication 11, dans lequel ladite partie de pont sur la paume (606) maintient lesdites plaques palmaire et dorsale à une distance définie l'une de l'autre.

13. Support orthopédique de poignet selon l'une quelconque des revendications précédentes, dans lequel ladite exostructure (600) entoure au moins partiellement la partie de l'anatomie à soutenir.

14. Support orthopédique de poignet selon l'une quelconque des revendications précédentes, dans lequel ladite exostructure (506, 530) a une partie d'avant-bras et une partie de main, ladite partie d'avant-bras comprenant une bande d'avant-bras radiale réglable (516, 520, 588) dans lequel la partie d'avant-bras peut être ajustée pour s'adapter aux avant-bras d'une variété d'utilisateurs différents.

15. Support orthopédique de poignet selon la revendication 14, dans lequel ladite bande d'avant-bras radiale réglable comprend une bande
- ayant un matériau d'attache à velcro (516) ;
- comprenant une première et une deuxième bande (588), ladite première bande ayant une pluralité de montants et ladite deuxième bande ayant une pluralité de trous pour recevoir lesdits montants ; ou
- est une bande de fermeture (520) fixée à ladite exostructure et une attache (524) fixée de manière réglable à ladite bande, ladite attache ayant une ouverture, ladite exostructure (506) ayant un crochet (528) auquel ladite attache est fixée.

16. Support orthopédique de poignet selon l'une quelconque des revendications précédentes, dans lequel ladite exostructure (600) comprend des plastiques de différentes densités.

17. Support orthopédique de poignet selon l'une quelconque des revendications précédentes, dans lequel l'épaisseur de ladite exostructure (600) n'est pas uniforme pour fournir différents niveaux de soutien en différents points sur le support.

18. Support orthopédique de poignet (590, 592) selon l'une quelconque des revendications précédentes, dans lequel ladite exostructure en plastique moulé comprend des première et deuxième pièces séparées.

19. Support orthopédique de poignet selon la revendication 18, dans lequel lesdites première et deuxième pièces séparées (594, 596) sont fixées l' une à l' autre par un procédé sélectionné parmi le soudage par ultrasons, les fermetures par pression (598a, 598b), le matériau d'attache à velcro, les rivets ou un adhésif.

20. Support orthopédique de poignet selon l'une quelconque des revendications précédentes, dans lequel ladite exostructure moulée (600) est directement moulée sur ledit tissu dans un moule.

21. Support orthopédique de poignet selon l'une quelconque des revendications précédentes, dans lequel le support à une ouverture de libération de la pression du canal carpien (529).

22. Support orthopédique de poignet selon l'une quelconque des revendications précédentes, dans lequel le support a une partie palmaire, avec un surmoule sur au moins une partie de la partie palmaire.
